# EUROPÄISCHE PATENTANMELDUNG

(11) **EP 1 321 107 A1**
(43) Veröffentlichungstag der Anmeldung: **25.06.2003**
(21) Anmeldenummer: 02028367.7
(22) Anmeldetag: 18.12.2002
(51) Int. Cl.: A61C 1/08, A61B 17/17

(54) **Positionierhilfe für chirurgische Werkzeuge**

(30) Priorität: 18.12.2001 DE 10162366
(71) Anmelder: Dr.-medic-stom./UMF Temeschburg, Herbert Hatzlhoffer, 76337 Waldbronn (DE)
(72) Erfinder: Dr.-medic-stom./UMF Temeschburg, Herbert Hatzlhoffer, 76337 Waldbronn (DE)
(74) Vertreter: Lemcke, Rupert, Dipl.-Ing.

(57) **Zusammenfassung**

Bei der erfindungsgemäßen Vorrichtung handelt es sich um eine in eine Schablone (15) integrierbare Positionierhilfe (1) für chirurgische Werkzeuge, insbesondere zur Bearbeitung von Kieferknochenmaterial (16). Zum Abstützen bzw. zum Ausrichten der Schablone im Bearbeitungsbereich ist die Positionierhilfe mit zumindest einem Distanzelement (2,12) kombinierbar. Im Bereich der Kieferchirurgie sollte das Distanzelement in etwa der Dicke der zu entfernenden Zahnfleischschicht entsprechen. Die Schablone kann dadurch im Arbeitsbereich trotz des fehlenden Zahnfleisches derart abgestützt werden, dass sie die zuvor am Modell bestimmte Ausrichtung erhält.

## Beschreibung

Die Erfindung betrifft eine in eine Schablone integrierbare Positionierhilfe für chirurgische Werkzeuge, insbesondere zur Bearbeitung von Kieferknochenmaterial.

Vor dem Einbringen von Zahnimplantaten müssen zunächst die Positionen festgelegt werden, die die Implantate im Kieferknochen einnehmen sollen. Dazu wird ein Abdruck von dem Bereich der Mundhöhle hergestellt, der die zahnlosen Stellen und gegebenenfalls dazu benachbarte Zähne bzw. Zahnreihen enthält. Von diesem Abdruck wird dann ein Modell hergestellt, das dem Bereich der Mundhöhle entspricht, in welchen die Implantate eingeführt werden sollen. An diesem Modell werden dann die Positionen der Implantate festgelegt. In einem nächsten Schritt wird eine Schablone für das Modell angefertigt. In diese Schablone werden an den Implantationsstellen Positionierhilfen eingebracht, die zur Führung der chirurgischen Werkzeuge bei der Knochenbearbeitung dienen. Bei den Positionierhilfen handelt es sich in der Regel um Hülsen. Das Festlegen der Hülsen in der Schablone geschieht meistens durch Eingießen.

Bevor Bohrungen am Kieferknochen vorgenommen werden können, muss das Zahnfleisch an den entsprechenden Stellen entfernt werden. In der Regel wird es dazu aufgeschnitten und weggeklappt. Danach wird die Schablone in den zu ihr passenden Mundbereich eingesetzt. Durch das Entfernen des Zahnfleisches hat sich jedoch der Mundbereich stark verändert. Die Schablone liegt nicht mehr lückenlos im entsprechenden Bearbeitungsbereich auf, da die Entfernung des Zahnfleisches zu Hohlräumen geführt hat. Bei sogenannten Freiendsituationen bzw. bei vollständiger Zahnlosigkeit (Endentation) kann dies zu erheblichen Problemen führen, da die Schablone nicht entsprechend ihrer gewünschten Lage ausgerichtet ist. Insbesondere während des Bearbeitungsvorganges selbst ist die vorgesehene Ausrichtung der Schablone nicht mehr gewährleistet, so dass es u.U. zu Fehlbohrungen kommt, d.h., dass die tatsächliche Bohrposition von der gewünschten Bohrposition bezüglich Lage und Winkel abweicht. Dies kann dazu führen, dass die Implantate nicht die gewünschten Positionen im Kieferknochen einnehmen.

Weiterhin können die Hohlräume zwischen Kieferknochenmaterial und Schablone dazu führen, dass während des Bearbeitungsvorganges die Schablone durch Beaufschlagung mit Kräften verformt wird. Die Schablone kann sich dabei beispielsweise nach unten durchbiegen oder in sich verdrehen.

Aufgabe der vorliegenden Erfindung ist es, eine Vorrichtung zur Verfügung zu stellen, mit der eine exakte Ausrichtung und Positionierung des Bearbeitungswerkzeugs möglich ist.

Diese Aufgabe wird erfindungsgemäß dadurch gelöst, dass die Positionierhilfe zum Abstützen und/oder Ausrichten der Schablone im Bearbeitungsbereich mit zumindest einem Distanzelement kombinierbar ist, das insbesondere dazu vorgesehen ist, sich im Bearbeitungsbereich am Kieferknochen abzustützen und damit die Schablone am Kieferknochen indirekt aufzulagern.

Bevor das Zahnfleisch an der entsprechenden Stelle der Mundhöhle entfernt wird, kann mit Hilfe eines Tiefenmessers die Zahnfleischdicke bestimmt werden. Die Positionierhilfen in der Schablone werden dann mit passenden Distanzelementen kombiniert, die in etwa der Dicke der zu entfernenden Zahnfleischschicht entsprechen. Die Schablone kann dadurch im Arbeitsbereich trotz des fehlenden Zahnfleisches derart abgestützt werden, dass sie die zuvor am Modell bestimmte Ausrichtung erhält. Durch die Abstützung der Schablone über die passenden Distanzelemente ist es nicht notwendig, während des eigentlichen Bearbeitungsvorganges auf die exakte Ausrichtung der Schablone zu achten, da die Schablone in ihrer Position exakt fixiert ist.

Die erfindungsgemäße Kombination von Positionierhilfe mit einem Distanzelement ist insbesondere bei der Bearbeitung von Freiendsituationen oder zahnlosen Kiefern von großem Vorteil, da eine definierte Abstützung der Schablone am Kieferknochen mit Hilfe der jeweiligen Distanzelemente möglich ist und dadurch eine Ausrichtung der Schablone entsprechend dem Modell vorgenommen wird.

Sobald die Schablone durch die erfindungsgemäße Abstützung in der Mundhöhle ausgerichtet ist, kann die Bearbeitung des Kieferknochens vorgenommen werden. In der Regel handelt es sich bei den Positionierhilfen um Hülsen, in denen das chirurgische Werkzeug geführt wird.

In einer besonders günstigen Ausführung der Erfindung wird die Positionierhilfe mit einem Distanzelement kombiniert, das seinerseits innen hohl ist. Das chirurgische Werkzeug wird dann sowohl durch die Positionierhilfe, als auch durch das Distanzelement geführt. Der Kanal in der Distanzhülse kann in seinen Abmessungen dem Kanal in der Positionierhilfe entsprechen. Es ist aber auch denkbar, dass der Kanal der Distanzhülse einen größeren oder einen kleineren Durchmesser im Vergleich zu dem Kanal der Positionierhilfe aufweist.

In einer vorteilhaften Ausführung der Erfindung werden die inneren Abmaße des Hohlraums im Distanzelement derart gewählt, dass das chirurgische Werkzeug entlang dessen Kanalwänden geführt wird. In diesem Fall tritt ein nur vernachlässigbares Spiel zwischen dem chirurgischen Werkzeug und dem Kanal im Inneren des Distanzelementes auf, so dass durch die Vorgabe der Führung ein seitliches Ausweichen während des Bearbeitungsvorganges ausgeschlossen ist.

Die erfindungsgemäße Kombination von Positionierhilfe mit einem Distanzelement kann auf unterschiedliche Arten und Weisen erfolgen. Beispielsweise kann das Distanzelement an die Positionierhilfe angeklebt werden. Es ist auch denkbar, dass das Distanzelement mit der Positionierhilfe durch Einrasten verbunden wird. Weiterhin ist es möglich, dass ein eigenes Halterungselement verwendet wird, welches das Distanzelement mit der Positionierhilfe verbindet. Prinzipiell können Distanzelement und Positionierhilfe lösbar oder unlösbar miteinander kombiniert werden. Im Falle einer lösbaren Verbindung bietet es sich aber vor allem an, das Distanzelement und die Positionierhilfe miteinander zu verschrauben. Hierzu kann die Positionierhilfe mit einem Innengewinde und das Distanzelement mit einem Außengewinde versehen sein. Das Distanzelement kann dann in die Positionierhilfe eingeschraubt werden.

Als besonders vorteilhaft hat es sich erwiesen, die Positionierhilfe nicht in die Schablone einzugießen, sondern in die Schablone einzuschrauben, wodurch beim Einbringen der Positionierhilfe nicht erst lange das Aushärten der Schablone abgewartet werden muss, sondern die Montage ohne Wartezeiten schnell fortgesetzt werden kann. Dazu kann die Positionierhilfe mit einem Außengewinde versehen werden. Es ist dabei günstig, wenn die Positionierhilfe zusätzlich einen Bereich aufweist, in den ein Imbusschlüssel, beispielsweise ein Innensechskant eingreifen kann, um die Positionierhilfe in die Schablone einzuschrauben.

Um mit dem Distanzelement einen exakt definierten Abstand vorzugeben, erweist es sich als besonders vorteilhaft, dass das Distanzelement bis zu einem Anschlag in die Positionierhilfe eingeschraubt bzw. bis zu einem Anschlag aus ihr herausgeschraubt werden kann. Es ist vorteilhaft, den Anschlag so zu wählen, dass wenn das Distanzelement an den Anschlag stößt, der dadurch definierte Abstand, der auszugleichenden Distanz zwischen Schablone und Kieferknochen entspricht.

Die erfindungsgemäße Kombination der Positionierhilfe mit einem Distanzelement kann auch in situ erfolgen, indem die Schablone in den zu bearbeitenden Kieferbereich eingesetzt wird und dann das Distanzelement bis zum Kieferknochenkontakt aus der Positionierhilfe herausgeschraubt wird. Durch entsprechendes Herausdrehen und Hineindrehen des Distanzelementes kann entweder nach Augenmaß oder nach exakten Lagevorgaben die Schablone im Bearbeitungsbereich kontinuierlich ausgerichtet und deren Lage im Kiefer stabilisiert werden.

Es ist auch denkbar, dass die Methode eines Einschraubens bis zum Anschlag und die Methode eines kontinuierlichen, in situ, Hinein- und Herausschraubens miteinander kombiniert werden. Diese Kombination erweist sich insbesondere bei einer Freiendsituation bzw. bei Einsetzung von Implantaten in einen zahnlosen Kiefer als besonders vorteilhaft.

Bei Freiendsituationen wird an der endständigen Implantatposition zunächst die Tiefe des Zahnfleisches gemessen. Entsprechend der Zahnfleischtiefe wird für diese endständige Positionierhilfe ein passendes Distanzelement mit Anschlag ausgewählt. Dieses Distanzelement wird bis zu seinem Anschlag in die endständige Positionierhilfe eingeschraubt. In einem nächsten Schritt wird dann das Zahnfleisch beim Patienten entfernt und die Schablone wird im Bearbeitungsbereich eingesetzt. Da die endständige Positionierhilfe bereits mit einem passenden Distanzelement zum Zahnfleischausgleich ausgestattet wurde, richtet sich die Schablone bei ihrem Einsatz in der Mundhöhle in die gewünschte Position aus. Die zwischen der endständigen Positionierhilfe und dem Zahn liegenden Positionierhilfen weisen jedoch noch einen Abstand zum Kieferknochen auf, da auch an diesen Stellen das Zahnfleisch entfernt wurde. Dieser Abstand wird mit Distanzelementen ausgeglichen, die kontinuierlich so weit aus der Positionierhilfe herausführbar sind, bis sie den Kieferknochen berühren. Dadurch wird die Schablone über ihren gesamten Bereich stabilisiert und es kann nicht zu Verformungen der Schablone während des Bohrvorganges kommen.

Zur Vorbereitung von Implantationen an zahnlosen Kiefern wird für den Einsatz der erfindungsgemäßen Positionierhilfen die Zahnfleischtiefe insgesamt an drei Stellen gemessen, in der Regel an den beiden endständigen Positionierhilfen und an einer mittleren Positionierhilfe. Passend zu den entsprechenden Zahnfleischtiefen wird für diese drei Positionierhilfen ein geeignetes Distanzelement ausgewählt und bis zum Anschlag in die jeweilige Positionierhilfe eingeschraubt. Dann wird das Zahnfleisch in dem Kieferbereich entfernt und die Schablone wird in den Arbeitsbereich eingesetzt. Die Schablone wird über die drei zuvor eingeschraubten Distanzelemente am Kieferknochen abgestützt und dadurch im Arbeitsbereich ausgerichtet. Die Schablone ist durch diese drei Auflagepunkte in ihrer Lage exakt fixiert. Die Positionierhilfen, die zwischen den mit Distanzelementen versehenen Positionierhilfen liegen weisen jedoch noch einen Abstand zum Kieferknochen auf, da auch an diesen Stellen das Zahnfleisch entfernt wurde. Dieser Abstand wird mit Distanzelementen ausgeglichen die so weit aus der Positionierhilfe herausschraubbar sind, bis sie den Kieferknochen berühren. Jetzt ist die Schablone in ihrem gesamten Bereich stabilisiert und es kann während des Bearbeitungsvorganges nicht zu Verformungen der Schablone kommen.

Insbesondere bei Implantationen an einem zahnlosen Kiefer, kann es unter Umständen vorteilhaft sein, die Schablone vor dem Bearbeitungsvorgang am Kiefer festzulegen. Dazu können durch die Positionierhilfen Schrauben in den Kiefer eingebracht werden. Die Schablone ist mit Hilfe dieser Schrauben im Bearbeitungsbereich festgelegt und fixiert. Ein Festlegen der Schablone am Kieferknochen erfolgt vorzugsweise über die endständigen bzw. mittleren Positionierhilfen.

Nachdem die Schablone erfindungsgemäß im Kieferbereich ausgerichtet ist, entweder nur lose aufliegend oder mit Schrauben festgelegt, kann der eigentliche Bearbeitungsvorgang am Knochen erfolgen. In der Regel wird dazu der Bohrer durch die Positionierhilfe und das angrenzende Distanzelement geführt und tritt dann in den Kieferknochen ein. Die Bearbeitungsposition und der Bearbeitungswinkel sind durch die Positionierhilfe und das Distanzelement genau vorgegeben. Als besonders günstig erweist es sich, wenn die Positionierhilfe und/oder das Distanzelement einen Anschlag für das chirurgische Werkzeug aufweist, mit dem zusätzlich die Bearbeitungstiefe vorgebbar ist. Bei der erfindungsgemäßen Vorrichtung ist der Abstand von dem Anschlag des chirurgischen Werkzeugs bis zum Kieferknochen genau vorgegeben. Die erfindungsgemäße Vorrichtung bietet daher den Vorteil, dass auch die Knochenbearbeitungstiefe mit Auswahl eines passenden Bohrers definiert vorgebbar ist. Der Bohrer muss so ausgewählt werden, dass er in seiner Länge der Knochenbearbeitungstiefe plus dem Abstand vom Werkzeuganschlag bis zum Eintritt in den Knochen spricht. Im Gegensatz zu den bei dem Stand der Technik verwendeten Bohrhülsen ist bei der erfindungsgemäßen Vorrichtung der Abstand zwischen Werkzeuganschlag und Knochenoberfläche bekannt. Mit Hilfe der erfindungsgemäßen Vorrichtung ist somit die Bohrung im Knochen sowohl bezüglich ihrer Lage als auch bezüglich ihrer Tiefe definiert vorgebbar. Fehlerquellen bei der Bearbeitung werden somit weitestgehend ausgeschlossen.

Die erfindungsgemäße Vorrichtung kann für unterschiedliche chirurgische Werkzeuge wie beispielsweise Knochenbohrer, Knochenfräser, Gewindeschneider usw. angewendet werden. Sie wird vorzugsweise im Bereich der Kieferchirurgie, insbesondere zur Präparation des Kieferknochens für den Einsatz von Implantaten eingesetzt. Prinzipiell ist die Idee aber auch auf andere Bereiche der Knochenchirurgie übertragbar.

Weitere Merkmale und Vorteile der Erfindung ergeben sich aus der Beschreibung von Ausführungsbeispielen anhand der Zeichnungen und aus den Zeichnungen selbst. Dabei zeigt:
- Figur 1: einen axialen Halbschnitt durch eine erfindungsgemäße Kombination einer Positionierhilfe mit einem Distanzelement, welches einen Anschlag aufweist;
- Figur 2: ein erfindungsgemäße Kombination einer Positionierhilfe mit einem Distanzelement, das keinen Anschlag hat;
- Figur 3: eine Positionierhilfe;
- Figur 4: ein Distanzelement mit Anschlag;
- Figur 5: ein Distanzelement ohne Anschlag;
- Figur 6: eine Positionierhilfe mit Distanzelementen ohne Anschlag und eine Positionierhilfe mit einem Distanzelement, welches einen Anschlag aufweist, in Kombination mit einer Freiendsituation.
- Figur 7: eine Positionierhilfe mit einem Distanzelement ohne Anschlag und Positionierhilfen mit Distanzelementen, die einen Anschlag aufweisen, in Kombination bei einer zahnlosen Situation.

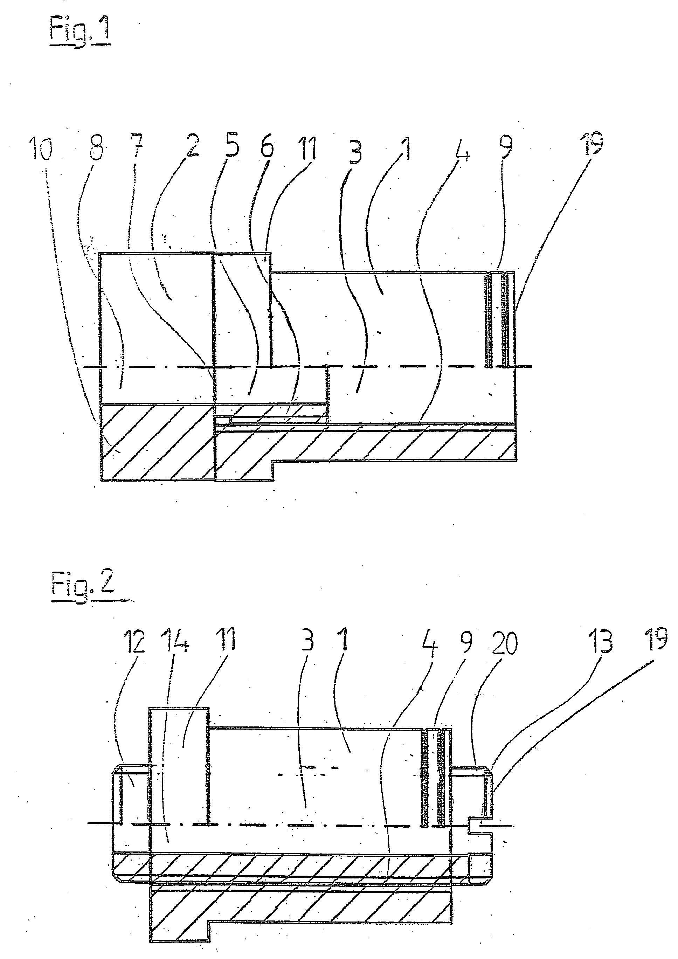

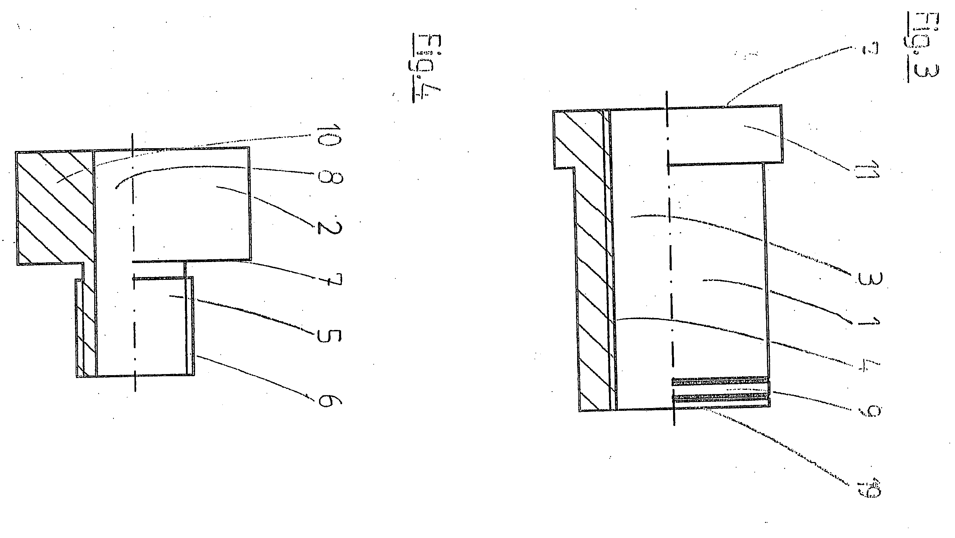

In Figur 1 ist die Kombination einer Positionierhilfe 1 mit einem Distanzelement 2 dargestellt. Die Positionierhilfe 1 weist eine Innenbohrung 3 auf. Die Bohrung 3 ist mit einem Innengewinde 4 versehen. Das Distanzelement 2 weist an seiner der Positionierhilfe 1 zugewandten Seite einen Zapfen 5 auf. Der Zapfen 5 ist mit einem Außengewinde 6 versehen. Das Distanzelement 2 wird in die Positionierhilfe 1 bis zu einem Anschlag 7 hineingeschraubt. Das Distanzelement 2 ist mit einer Innenbohrung 8 versehen, die in ihrem Durchmesser kleiner ist als die Innenbohrung 3 der Positionierhilfe 1. Die Positionierhilfe 1 wird in die Schablone 15 durch Eingießen integriert. Zum besseren (insbesondere formschlüssigen) Halt beim Integrieren in die Schablone 15 ist die Positionierhilfe 1 mit Riffeln 9 versehen. Der Distanzkörper 10 des Distanzelementes 2 entspricht in etwa der Dicke des Zahnfleisches an der Stelle der Positionierhilfe 1. Die Positionierhilfe 1 ist mit einem Fuß 11 versehen.

In Figur 2 ist eine Positionierhilfe 1 mit einem Distanzelement 12 kombiniert, welches keinen Anschlag aufweist. Das Distanzelement 12 ist mit einem Außengewinde 20 versehen. Um das Distanzelement 12 in die Positionierhilfe 1 hineinzuschrauben bzw. herauszuschrauben, ist das Distanzelement 12 mit Schlitzen 13 ausgestattet, in die ein Schraubenzieher eingreifen kann. Das Distanzelement 12 wird bei seinem Einsatz so weit aus der Positionierhilfe herausgedreht, dass es Kontakt zum Kieferknochen 16 hat. Die Lücke 17, die sich nach dem Entfernen des Zahnfleisches ergeben hat, wird somit von dem Distanzelement überbrückt. Die in Figur 2 dargestellte Positionierhilfe 1 entspricht der bereits in Figur 1 beschriebenen Positionierhilfe 1.

In Figur 3 ist die Positionierhilfe 1 noch einmal getrennt dargestellt.

In Figur 4 ist das Distanzelement 2 (mit Anschlag) einzeln dargestellt. Die Dicke des Distanzkörpers 10 wird entsprechend der zu überbrückenden Lücke zwischen Positionierhilfe 1 und Kieferknochen 16 ausgewählt. Die Abstufung kann beliebig vorgenommen werden beispielsweise in 1 mm-Schritten.

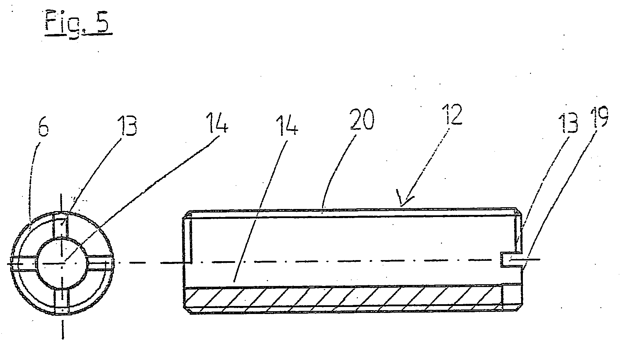

In Figur 5 findet sich eine separate Darstellung des Distanzelementes 12 ohne Anschlag, einmal als axialer Halbschnitt und einmal als Vorderansicht. Dieses Distanzelement 12 wird so weit aus der Positionierhilfe 1 herausgeschraubt, bis der Abstand zwischen Schablone und Knochen 16 überbrückt ist. Das Distanzelement 12 hat ebenfalls eine Innenbohrung 14, in der das chirurgische Werkzeug geführt werden kann. Die Innenbohrung 14 des Distanzelementes 12 hat im Ausführungsbeispiel einen kleineren Durchmesser als die Innenbohrung 3 der Positionierhilfe 1. Eine Führung des chirurgischen Werkzeugs findet daher in erster Linie durch das Distanzelement 12 statt.

Auch die Innenbohrung 8 des Distanzelementes 2 (mit Anschlag) hat einen kleineren Durchmesser als die Innenbohrung 3 der Positionierhilfe 1. Somit dient auch im Fall einer Kombination des Distanzelementes 2 (mit Anschlag) und der Positionierhilfe 1 in erster Linie die Innenbohrung 8 des Distanzelementes 2 zur Führung des chirurgischen Werkzeugs.

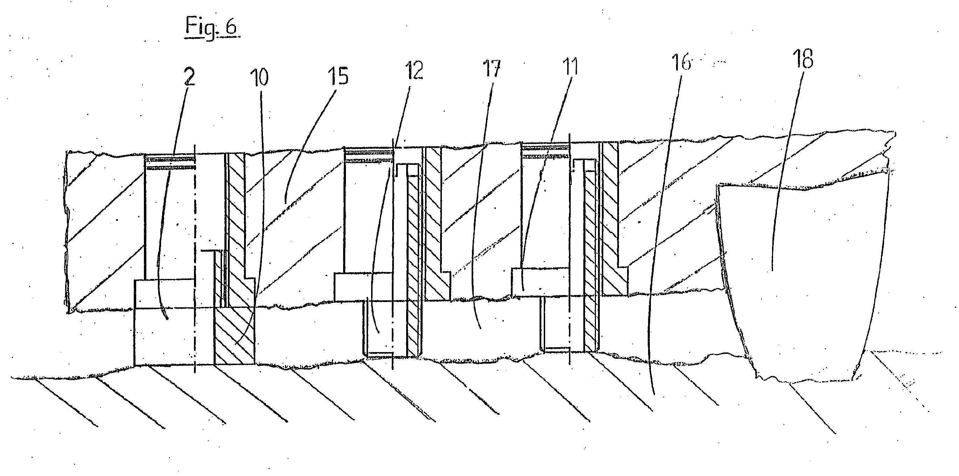

In Figur 6 wird der Einsatz der erfindungsgemäßen Vorrichtung bei einer Freiendsituation dargestellt. Dabei kommen eine Positionierhilfe 1 mit einem Distanzelement 2 (mit Anschlag) und Positionierhilfen 1 mit Distanzelementen 12 (ohne Anschlag) in Kombination zum Einsatz. Die Positionierhilfen 1 sind in eine Schablone 15 integriert. Die Positionierhilfen 1 schließen in ihrem Fuß 11 bündig mit der Schablone 15 ab. Zwischen der Schablone 15 und dem Kieferknochen 16 ist durch das Entfernen des Zahnfleisches ein Hohlraum 17 entstanden. Dieser Hohlraum 17 wird mit den Distanzelementen 2, 12 überbrückt. Dabei dient das Distanzelement 2 in erster Linie zum exakten Ausrichten der Schablone 15 im Bearbeitungsbereich. Der Distanzkörper 10 des Distanzelementes 2 wird entsprechend den Abmessungen des Hohlraumes 17 ausgewählt. Die Distanzelemente 12 dienen der zusätzlichen Abstützung der Schablone 15. Sie werden so weit aus der Positionierhilfe 1 herausgeschraubt, bis sie in Kontakt mit dem Kieferknochen 16 gelangen und sich so an diesen abstützen.

Bei der in Figur 6 dargestellten Freiendsituation liegt die Schablone 15 an ihrem einen Ende auf einem Zahn 18 auf. Ohne das mit der Positionierhilfe 1 kombinierte Distanzelement 2 wäre die Schablone 15 nicht in ihrer Lage fixiert. Sie würde im Bearbeitungsbereich wackeln. Durch das Distanzelement 2 wird die Schablone im Arbeitsbereich definiert ausgerichtet und zwar hinsichtlich des Abstands zum Kieferknochen und auch des Winkels, den die Achse der Positionierhilfe und damit auch die Achse des Implantats gegenüber dem Kieferknochen aufweist.

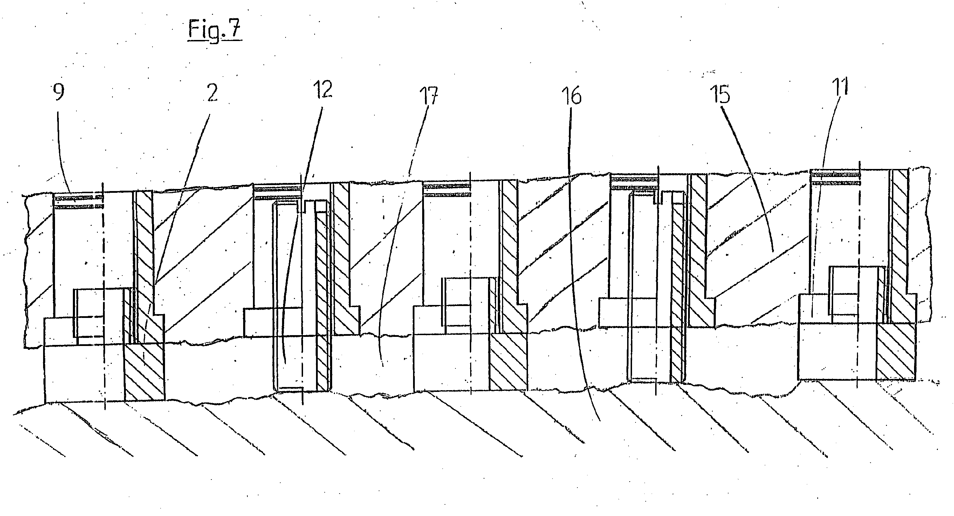

In Figur 7 ist der Einsatz der erfindungsgemäßen Vorrichtung bei einer zahnlosen Situation (Endentation) dargestellt. Die Schablone 15 hat im Gegensatz zur Freiendsituation keinen Zahn als Auflagepunkt. Die Schablone 15 wird am Kieferknochen 16 mit den beiden endständigen Distanzelementen 2 abgestützt. Zusätzlich ist noch in der Mitte ein Distanzelement 2 (mit Anschlag) eingebracht. Zwischen den Distanzelementen 2 (mit Anschlag) können Distanzelemente 12 (ohne Anschlag) in die Positionierhilfen 1 eingeführt werden.

## Patentansprüche

1. In eine Schablone (15) integrierbare Positionierhilfe (1) für chirurgische Werkzeuge, insbesondere zur Bearbeitung von Kieferknochenmaterial (16) **dadurch gekennzeichnet,**
**dass** die Positionierhilfe (1) zum Abstützen und/oder Ausrichten der Schablone (15) im Bearbeitungsbereich mit zumindest einem Distanzelement (2, 12) kombinierbar ist.

2. Positionierhilfe nach Anspruch 1,
**dadurch gekennzeichnet,**
**dass** das Distanzelement (2, 12) innen hohl ist und das chirurgische Werkzeug durch das Distanzelement (2, 12) bewegbar ist.

3. Positionierhilfe nach Anspruch 2,
**dadurch gekennzeichnet,**
**dass** das chirurgische Werkzeug in der Innenbohrung (8, 14) des Distanzelementes (2, 12) führbar ist.

4. Positionierhilfe nach Anspruch 1,
**dadurch gekennzeichnet,**
**dass** das Distanzelement (2, 12) und die Positionierhilfe (1) durch Verschrauben aneinander festlegbar sind.

5. Positionierhilfe nach Anspruch 1,
**dadurch gekennzeichnet,**
**dass** das Distanzelement (2, 12) und die Positionierhilfe (1) durch Verkleben kombinierbar sind.

6. Positionierhilfe nach Anspruch 1,
**dadurch gekennzeichnet,**
**dass** das Distanzelement (2, 12) und die Positionierhilfe (1) durch Einrasten kombinierbar sind.

7. Positionierhilfe nach Anspruch 1,
**dadurch gekennzeichnet,**
**dass** die Positionierhilfe (1) mit einem Innengewinde (4) und das Distanzelement (2, 12) mit einem Außengewinde (6) versehen ist.

8. Positionierhilfe nach Anspruch 1,
**dadurch gekennzeichnet,**
**dass** das Distanzelement (2, 12) stufenlos in die Positionierhilfe (1) einführbar und/oder aus ihr herausführbar ist und dadurch ein Abstützen und/oder Ausgleichen der Schablone (15) in situ ermöglicht wird.

9. Positionierhilfe nach Anspruch 1,
**dadurch gekennzeichnet,**
**dass** das Distanzelement (2, 12) bis zu einem Anschlag (7) in die Positionierhilfe (1) einführbar und/oder bis zu einem Anschlag (7) aus der Positionierhilfe (1) herausführbar ist und dadurch eine abstandsdefinierte Abstützung möglich ist.

10. Positionierhilfe nach Anspruch 1,
**dadurch gekennzeichnet,**
**dass** die Schablone (15) über die Positionierhilfe (1) im Bearbeitungsbereich festlegbar ist.

11. Positionierhilfe nach Anspruch 1,
**dadurch gekennzeichnet,**
**dass** das chirurgische Werkzeug bis zu einem Anschlag (19) an der Positionierhilfe (1) und/oder am Distanzelement (12) bewegbar ist und dadurch die Tiefe des Bearbeitungsvorganges definiert vorgebbar ist.

12. Positionierhilfe nach Anspruch 1,
**dadurch gekennzeichnet,**
**dass** die Positionierhilfe (1) zum Einschrauben in die Schablone (15) mit einem Außengewinde versehen ist.
